# EUROPEAN PATENT APPLICATION

(11) **EP 4 763 349 A1**
(43) Date of publication of application: **24.06.2026**
(21) Application number: 25225150.9
(22) Date of filing: 18.12.2025
(51) Int. Cl.: B05B 11/02, B05B 11/04, B05B 11/10, B65D 47/18

(54) **METERED-DOSE AIRLESS DISPENSING POUCH WITH INTERNAL BULB PUMP**

(30) Priority: 18.12.2024 US 202463735433 P
(71) Applicant: 1Touch Holdings, Inc., Farmington, Connecticut 06032 (US)
(72) Inventor: CURTISS, Charles, Norwalk, CT 06855 (US)
(74) Representative: Office Freylinger

(57) **Abstract**

Dispensing assembly (100), comprising a flexible, collapsible pouch (102) defining a reservoir configured to contain a liquid; a rigid canoe (104) sealed to the pouch and extending into an interior of the pouch; a squeezable bulb (106) disposed at least partially within the pouch and supported by the canoe structure, the bulb defining a dosing chamber; a first one-way valve (220) fluidly connecting the reservoir to the dosing chamber; a second one-way valve (320) fluidly connecting the dosing chamber to an outlet; and an outlet tip in fluid communication with the second one-way valve; wherein the pouch and the squeezable bulb are configured such that application of external pressure through the pouch collapses the squeezable bulb to expel a metered dose of liquid from the dosing chamber (206).

## Description

### CROSS REFERENCE TO RELATED APPLICATIONS

This Application claims priority to U.S. Provisional Patent No. 63/735,433 filed on December 18, 2024, the entire contents of which are incorporated by reference.

### FIELD OF THE INVENTION

The present disclosure relates generally to dispensing packages and, more particularly, to flexible pouches equipped with internal pump mechanisms configured to dispense metered doses of a liquid or other flowable composition in an airless manner.

### BACKGROUND

Metered-dose dispensers are widely used for ophthalmic medications, otic medications, animal care products, personal care products, and technical fluids. Current commercially available dropper bottles and pouches typically rely on rigid or semi-rigid containers with external actuators such as buttons or squeeze bulbs. Many such packages entrain air into the container during use, which can compromise sterility and stability of sensitive formulations.

Flexible pouches with fitments at one end are also known. In some constructions, a pump or valve is mounted externally to the pouch or integrated into a fitment accessible outside the pouch. These arrangements generally use multiple seals, complex die-cut parts, tight tolerances, and precise assembly operations, which increase cost and introduce multiple failure points. Some external button-based designs can also use relatively high actuation forces and may not provide consistent drop volume.

There remains a need for a simple, low-cost, airless dispensing system that can be integrated into a flexible pouch and that reliably delivers a metered dose with each actuation, while minimizing the number of seals and components, and optionally providing inherent child resistance by locating the actuator inside an opaque pouch.

### SUMMARY

Disclosed herein is a dispensing assembly, comprising a flexible, collapsible pouch defining a reservoir configured to contain a liquid; a rigid canoe sealed to the pouch and extending into an interior of the pouch; a squeezable bulb disposed at least partially within the pouch and supported by the canoe structure, the bulb defining a dosing chamber; a first one-way valve fluidly connecting the reservoir to the dosing chamber; a second one-way valve fluidly connecting the dosing chamber to an outlet; and an outlet tip in fluid communication with the second one-way valve; wherein the pouch and the squeezable bulb are configured such that application of external pressure through the pouch collapses the squeezable bulb to expel a metered dose of liquid from the dosing chamber through the second one-way valve and the outlet tip, and release of the external pressure allows the bulb to expand and refill the dosing chamber from the reservoir through the first one-way valve while the second one-way valve remains closed, thereby maintaining an airless condition within the pouch.

Disclosed herein too is a method of dispensing a liquid, comprising providing a dispensing assembly according to claim 1, the pouch being pre-filled with a liquid in the reservoir; applying external pressure to a region of the pouch overlying the squeezable bulb to collapse the bulb against an internal stem member, thereby generating positive pressure in the dosing chamber and opening the second one-way valve while closing the first one-way valve, so as to expel substantially one metered dose of the liquid from the outlet tip; and releasing the external pressure to allow the bulb to expand, thereby generating negative pressure in the dosing chamber and opening the first one-way valve while closing the second one-way valve, so as to refill the dosing chamber from the reservoir while preventing air ingress through the outlet tip.

Disclosed herein too is a system for delivering metered doses of a liquid, comprising a dispensing assembly pre-filled with a liquid selected from a medication, a lubricant, a biological fluid, a pharmaceutical liquid, an aqueous fluid, an organic fluid, or a combination thereof; and instructions indicating that the user is to apply external pressure to a specified region of the pouch to deliver one metered drop per actuation until the pouch collapses.

### BRIEF DESCRIPTION OF THE FIGURES

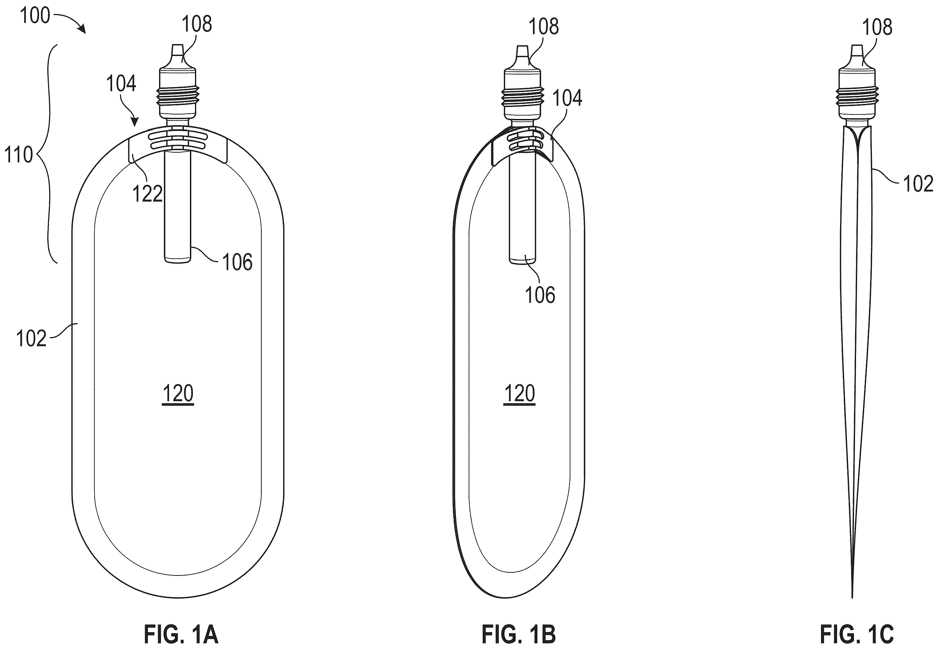

FIGS. 1A, 1B and 1C comprises a dispensing assembly comprises a flexible pouch that encapsulates a primary reservoir (that is filled with a fluid that is to be dispensed) within which is present a portion of a pump assembly. The bulb assembly contains (from bottom-to-top) a bulb assembly, a canoe (also referred to herein as a winged fitment that anchors and aligns the bulb assembly along with its valves) that provides a seal to the flexible pouch and shapes how the flexible pouch collapses when it is squeezed and a nozzle assembly through which a metered quantity of a fluid from the flexible pouch may be discharged;
FIGS. 2A, 2B and 2C are depictions of an exemplary manner in which the pump assembly is inserted into the flexible pouch with the canoe (over which the pouch is draped and heat sealed);
FIG. 3 depicts the pump assembly along with cap;
FIGS. 4A and 4B are also used to depict the pump assembly; and
FIGS. 5A and 5B schematically illustrates the overall flow circuit.

### DETAILED DESCRIPTION

Disclosed herein is a dispensing assembly that includes a flexible pouch disposed over a pump assembly that comprises two one-way valves. The two one-way valves operate cooperatively with one-another to permit the repeated discharge of a metered dose of a fluid from the flexible pouch to a recipient. The two one-way valves are never both simultaneously opened or closed during the operation of the dispensing assembly. When one of the two one-way valves is opened the other is closed and vice-versa. When an external pressure is applied at a designated position on the dispensing assembly, one of the two-way valves opens to permit a certain volume of fluid to flow from the flexible pouch into an internal stem (hereinafter stem), while the other two way valve is simultaneously closed, thus preventing external air from entering into the dispensing assembly and mixing with the fluid. When the dispensing assembly is depressed a second time, the volume of fluid contained in the stem is ejected through one of the one-way valves, while the other one-way valve is closed until the external pressure reaches a stop (on the stem) at which point the closed valve opens and the open valve closes to refill the stem. In other words, the two one-way valves are never simultaneously both open or both closed.

The pump assembly includes an elastomeric squeeze bulb (which functions as an actuator) defining an internal dosing chamber. The bulb may be generally bulbous or cylindrical and is formed from a flexible material that can be compressed and allowed to recover elastically. The stem is disposed within the bulb and extends toward an interior wall of the bulb. The stem and the bulb wall cooperate to limit the maximum collapse of the bulb in response to external pressure. By selecting the geometry of the bulb and the stem, the maximum volumetric displacement of the dosing chamber, and thus the dose size, can be set for a given design. For example, a 30 microliter dose can be provided with repeatability within a few microliters when operated by a user.

The dispensing assemblies disclosed herein provide numerous advantages over conventional pump-based or bottle-based dosing systems. In particular, locating the bulb entirely within a flexible, collapsible pouch enables airless dispensing, thereby minimizing or eliminating ingress of ambient air that could compromise sterility, oxidation stability, or shelf life of sensitive formulations. The internal placement of the actuator further reduces the number of external seals and interfaces, lowering manufacturing complexity and decreasing potential leak paths or failure points. By employing two one-way valves arranged in series and a bulb whose collapse-stroke is physically limited by an internal stop structure, the assemblies deliver a highly repeatable metered dose independent of user squeezing force, resulting in superior dose accuracy relative to conventional squeeze bottles.

With reference now to FIGS. 1A, 1B and 1C, a dispensing assembly 100 comprises a flexible pouch 102 (hereinafter pouch 102 or flexible pouch 102) that encapsulates a primary reservoir (that is filled with a fluid that is to be dispensed) within which is present a portion of a pump assembly 110 that contains (from bottom-to-top) a bulb assembly 106, a canoe 104 (also referred to herein as a winged fitment that anchors and aligns the bulb assembly 106 along with its valves) that provides a seal to the flexible pouch 102 and shapes how the flexible pouch 102 collapses when it is squeezed, and a nozzle assembly 108 through which a metered quantity of a fluid from the flexible pouch 102 may be discharged. FIGS. 1A, 1B and 1C are each side views of the dispensing assembly 100. FIG. 1Ais a face-on side view while FIG. 1C is an edge-on side view. FIG. 1B is an intermediate side view that lies between the face-on side view and the edge-on side view.

The flexible pouch 102 is collapsable and serves as the primary reservoir (i.e., the fixed volume) 120 for containing the liquid formulation that is discharged from the dispensing assembly. The internal reservoir 120 that is pre-filled at manufacture with a liquid product, such as an ophthalmic medication, an otic medication, a veterinary medication, a nutraceutical liquid, or a non-medical liquid such as lubricant or cleaning fluid.

The pouch may be fabricated from one or more polymeric films, laminates, or multilayer barrier structures, such as a polymer-containing laminate, a metallized film, a biodegradable film, or any combination thereof, selected to provide appropriate mechanical strength, puncture resistance, moisture barrier performance, and chemical compatibility with the contained liquid. In a preferred embodiment, the flexible pouch 102 is constructed from films thin enough to allow predictable manual deformation under squeezing forces (provided by a human hand without mechanical assistance), yet robust enough to withstand filling, sealing, sterilization, and distribution without rupture. Because the flexible pouch 102 collapses as liquid is dispensed, it permits airless operation by preventing air from being drawn into the internal pump assembly, thereby maintaining the integrity and sterility of the liquid product. The flexible pouch 102 may be pre-sterilized or sterilized post-fill, depending on regulatory requirements for the medication or fluid contained within.

The flexible pouch 102 is configured to interface mechanically and fluidically with the canoe 104 that houses the internal pump assembly 110. A perimeter region of the pouch is heat-sealed, ultrasonically welded, or otherwise bonded to a flange of the canoe 104, forming a hermetic seal that isolates the internal reservoir from the external environment. During use, the pouch 102 drapes over the canoe's 104 contoured upper surface and extends downward around its sides, ensuring that external squeezing forces applied to the pouch are transmitted efficiently to the internal bulb of the bulb assembly 106 in a consistent manner. As liquid is dispensed, the pouch 102 undergoes controlled volumetric collapse, conforming around the canoe 104 and the bulb assembly 106 to maintain constant negative pressure behavior during refill cycles. This controlled collapse enables high evacuation efficiency, allowing nearly all of the stored liquid to be delivered as metered doses with minimal residual volume remaining. In certain embodiments, the pouch may include printed indicia, transparency windows, or tactile features that guide the user to the appropriate squeeze zone while preserving the pouch's flexibility and hermetic integrity.

As noted above, the flexible pouch 102 may comprise a polymer film or a polymer laminate. The polymer film or polymer laminate may be of a strength effective to collapse under atmospheric pressure as liquid is dispensed from the flexible pouch 102. The polymer laminate may comprise two or more layers of polymer film. The laminate is preferably moisture and/or oxygen impermeable. It is thermally stable and does not undergo oxidation or ozonization when exposed to the atmosphere.

The polymer film or the polymer laminate may comprise an organic polymer that is selected from a wide variety of thermoplastic polymers, blend of thermoplastic polymers, thermosetting polymers, or blends of thermoplastic polymers with thermosetting polymers. The organic polymer may also be a blend of polymers, copolymers, terpolymers, or combinations comprising at least one of the foregoing organic polymers. The organic polymer can also be an oligomer, a homopolymer, a copolymer, a block copolymer, an alternating block copolymer, a random polymer, a random copolymer, a random block copolymer, a graft copolymer, a star block copolymer, a dendrimer, a polyelectrolyte (polymers that have some repeat groups that contain electrolytes), a polyampholyte (a polyelectrolyte having both cationic and anionic repeat groups), an ionomer, or the like, or a combination comprising at last one of the foregoing organic polymers. The organic polymers have a number average molecular weights greater than 10,000 grams per mole, preferably greater than 20,000 g/mole and more preferably greater than 50,000 g/mole.

Examples of thermoplastic polymers include a polyacrylic, a polycarbonate, a polyalkyd, a polystyrene, a polyolefin, a polyester, a polyamide, a polyaramid, a polyamideimide, a polyarylate, a polyacrylate, a polyurethane, an epoxy, a phenolic, a polysiloxane, a polyarylsulfone, a polyethersulfone, a polyphenylene sulfide, a polysulfone, a polyimide, a polyetherimide, a polytetrafluoroethylene, a polyetherketone, a polyether ether ketone, a polyether ketone ketone, a polybenzoxazole, a polyoxadiazole, a polybenzothiazole, a polycarbonate, a polyacetal, a polyanhydride, a polyvinyl ether, a polysulfonate, a polysulfide, a polythioester, a polysulfone, a polysulfonamide, a polyurea, a polyphosphazene, a polysilazane, a polyolefin, or the like, or a combination thereof.

Examples of thermosetting polymers include epoxy polymers, unsaturated polyester polymers, polyimide polymers, bismaleimide polymers, bismaleimide triazine polymers, cyanate ester polymers, vinyl polymers, benzoxazine polymers, benzocyclobutene polymers, polyacrylics, polyalkyds, phenol-formaldehyde polymers, novolacs, resoles, melamine-formaldehyde polymers, urea-formaldehyde polymers, hydroxymethylfuran polymers, polyisocyanates, or the like, or a combination comprising at least one of the foregoing thermosetting polymers.

A preferred polymer for use in the flexible pouch 102 is polyester, polyethylene, polypropylene, polyamide, or a combination thereof. Polymeric laminates used in the flexible pouch 102 include high density polyethylene in one layer of the laminate and a polyamide in another layer of the polyamide.

Examples of metallized films that may be used in the flexible pouch 102 selected from metallized polyester, metallized oriented polypropylene, metallized nylon, metallized polyethylene, aluminum-foil laminates, or metal-oxide coated polymer films, or combinations thereof, configured to provide enhanced barrier properties, mechanical durability, and chemical compatibility with the contained liquid. The metal used in the metallized film is aluminum.

Examples of biodegradable materials that may be used in the flexible pouch 102 include polylactic-glycolic acid (PLGA), poly-caprolactone (PCL), copolymers of polylactic-glycolic acid and poly-caprolactone (PCL-PLGA copolymer), polyhydroxy-butyrate-valerate (PHBV), polyorthoester (POE), polyethylene oxide-butylene terephthalate (PEO-PBTP), poly-D,L-lactic acid-p-dioxanone-polyethylene glycol block copolymer (PLA-DX-PEG), or a combination thereof. Example of biological polymers include naturally derived polymers (alginate, hyaluronic acid, chitosan, heparin, cellulose ethers (e.g. carboxymethyl cellulose, cellulose), elastin, gelatin, starch, carob gum, pectin, guar gum, carrageenan collagen, xanthan gum, fibronectin, elastin, albumin, lignin, glycosaminoglycans, chitin (including nanofibril form), or the like, or a combination thereof.

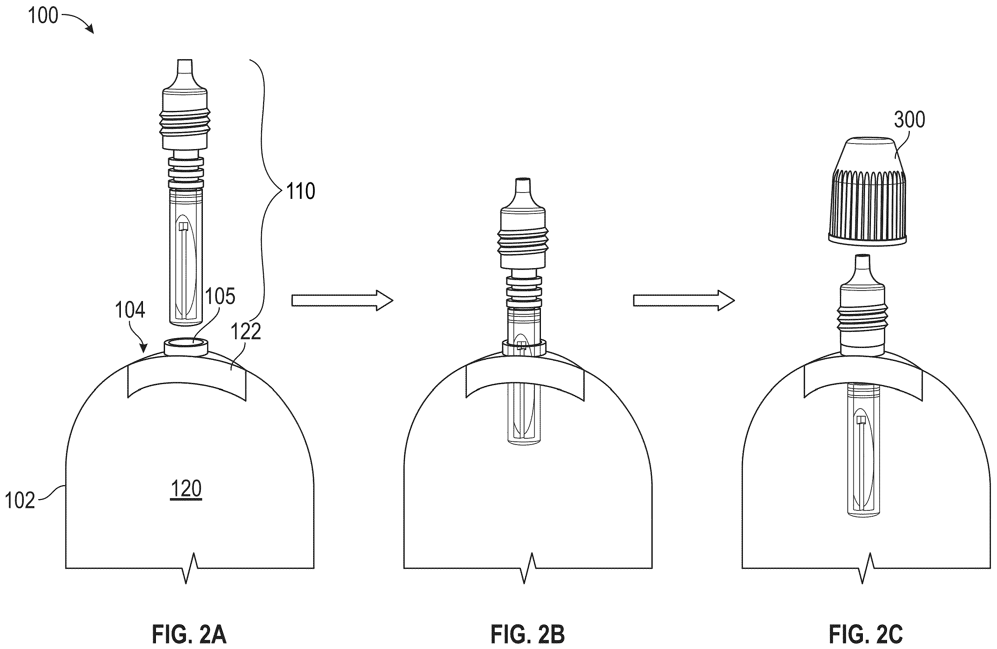

With reference again to FIGS. 1A, 1B, and 1C, the flexible pouch 102 is depicted as interfacing with the canoe 104 that houses the internal pump assembly 110. FIGS. 2A and 2B show an expanded view of the pump assembly 110 as well as the nozzle 108 (which protrudes from the pump assembly). The nozzle 108 lies outside the flexible pouch 102 and is mounted at one end of the pump assembly 110.

The canoe 104 is a rigid canoe structure or canoe fitment and is sealed to an opening (not shown) in the flexible pouch 102. In one embodiment, the canoe 104 is a molded polymer component having a generally elongate, concave profile resembling a canoe, with side walls and ribs that provide structural rigidity. The molded polymer component is preferably one that has a glass transition temperature or a melting point greater than room temperature. Some of the thermoplastic polymers listed above, with glass transition temperatures or melting point temperatures greater than 75°C, may be used to form the canoe 104.

The canoe 104 performs several important functions - (a) It provides a rigid interface via an external flange 122 that can be heat-sealed to the flexible pouch film; (b) it defines an internal recess or cradle 123 in which the pump assembly 110 is housed and supported, with ribs or other internal features that properly orient the bulb 206, stem member 214, and outlet module 208. It serves as a filling interface for manufacturing equipment (in an industrial facility) that is used to manufacture the dispensing assembly 100. In certain embodiments, the canoe 104 is configured so that industrial filling lines can insert a fill nozzle into the canoe opening and fill the pouch 102 through the canoe end, after which the pump assembly 110 is installed and locked in place. The canoe 104 facilitates the creation of a predictable contact region for the flexible pouch 102. The pouch film rests over the top of the canoe 104 and drapes around it, so that when pressure is applied externally to the region corresponding to the bulb 206, the force is transmitted consistently to the bulb for reliable actuation.

FIGS. 2A, 2B and 2C are depictions of an exemplary manner in which the pump assembly 110 is inserted into the flexible pouch 102 with the canoe 104 (over which the pouch 102 is draped and heat sealed.

The flexible pouch 102 may be heat-sealed to an external flange 122 of the canoe 104, so that the canoe structure projects into the interior of the pouch and supports internal components (i.e., components of the pump assembly110). The flexible pouch 102 drapes over the upper surface of the canoe 104 and extends downward around the sides of the canoe 104, as schematically indicated in FIGS. 2A, 2B and 2C.

FIG. 2A depicts the flexible pouch 102 that is sealed to the canoe 104. The canoe 104 has flanges 122 to which the flexible pouch 102 is heat sealed to. The pump assembly 110 (which will be described in detail later) is reversibly inserted through a port 105 in the canoe 104. The pump assembly 110 can be inserted into the flexible pouch 102 via port 105 and can also be removed from the flexible pouch 102 via port 105. FIG. 2B depicts the pump assembly 110 after it is inserted into the flexible pouch 102. Port 105 can be used to fill the flexible pouch 102 with a desired fluid prior to assembling the pump assembly 110 and capping the dispensing assembly with a cap 300 (see FIG. 2C). Arrows in the FIG. 2A, 2B and 2C depict the sequence of events that bring about the assembly of the dispensing assembly 100. In a preferred embodiment, the pump assembly 110 is a snap-fit or friction-fit together, such that no adhesive or welding is needed to assemble the internal components. The only permanent seal in the assembly is the heat seal between the canoe 104 and the flexible pouch 102, thereby simplifying manufacturing and reducing failure points.

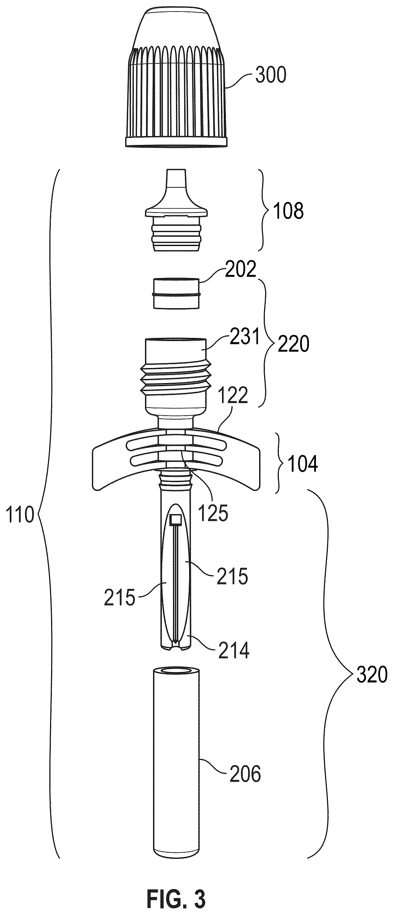

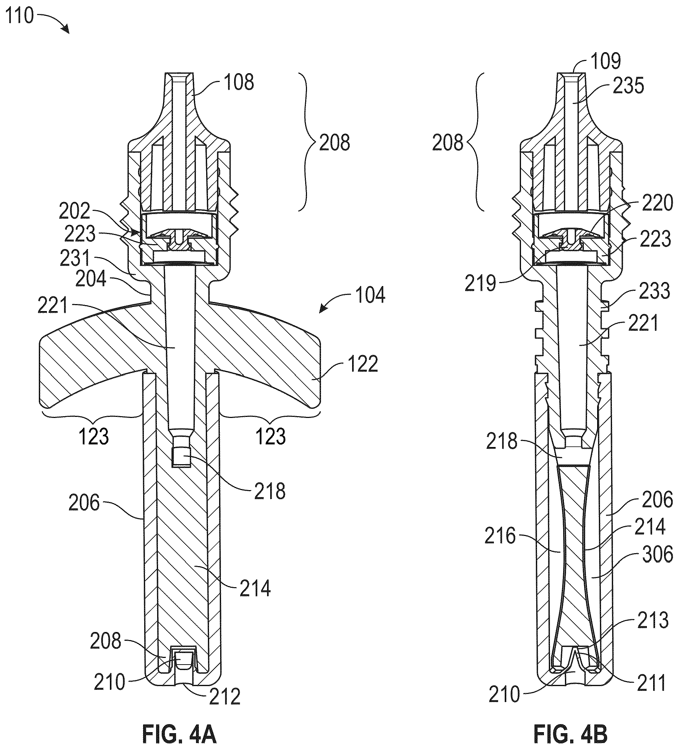

FIG. 3 depicts the pump assembly 110 along with cap 300. FIGS. 4A and 4B are also used to depict the pump assembly 110. The pump assembly 110 comprises two valves - an inlet one-way valve 320 and an outlet one-way valve 220. As seen previously in the FIGS. 2A, 2B and 2C, the pump assembly 110 is inserted into the flexible pouch 102 via port 105. The outlet one way valve 220 is in fluid communication with an outlet tip or nozzle 108. The nozzle 108 has an outlet port (a dropper orifice) 109 through which the liquid stored in the reservoir 120 may be discharged in a metered fashion.

The inlet one-way valve 320 is located at one end of the pump assembly 110, while the outlet one-way valve 220 is located at the opposing end of the pump assembly 110. The inlet one-way valve 320 comprises a squeezable bulb 206 that is disposed on an internal stem member 214. The internal stem member 214 has a profile that approximates two opposed prongs 215 of a tuning fork, with a contoured surface that lies opposite an inner wall of the squeezable bulb 206. The opposed prongs 215 have a concave region at their center that permits a suction to be created when the bulb 206 is squeezed. When external pressure is applied through the flexible pouch 102 (see FIG. 1A - 1C) to squeeze the squeezable bulb 206, the bulb wall collapses against the stem member 214, and the range of travel of the wall is mechanically limited by contact with the stem 214. This limitation defines a reproducible maximum collapse volume, thereby defining a predetermined dosing volume that is eventually ejected through the nozzle 108.

The stem member 214 may be formed integrally with a rigid frame or may be a separate insert that snaps into the canoe 104 or other support structure. By changing the size, shape, or position of the stem member 214, or by changing the size and thickness of the bulb 206, the metered dose volume can readily be changed (e.g., from about 10 microliters to about 60 microliters or more) without changing the architecture of the dispensing assembly.

In an embodiment, the stem member 214 may comprise the same polymeric material as that used in the canoe 104. The stem member is generally manufactured from a material that does not interact with the fluid that is to be discharged. The material is preferably a rigid polymer that has a glass transition and/or a melting temperature that is greater than or equal to 50°C, preferably greater than or equal to 70°C, preferably greater than or equal to 90°C, and more preferably greater than 100°C. Some of the thermoplastic or thermosetting polymers listed above may be used in the stem member 214.

With reference now to FIG. 4A and 4B, the bulb 206 defines an internal dosing chamber 221. In one preferred embodiment, the bulb 206 includes at its lower end an integral duckbill valve 210. The duckbill valve 210 comprises a pair of opposed, resilient lips 211 that meet along a slit 213. When there is negative pressure inside the bulb relative to the flexible pouch reservoir 120 (See FIG. 1A), the lips 211 elastically separate, permitting liquid to flow from the pouch 102 into the dosing chamber 221. When positive pressure is applied to the dosing chamber 206, the lips 211 of the duckbill valve 210 are pressed together more tightly, preventing backflow toward the reservoir. Thus, the duckbill valve functions as a first one-way check valve 320 (See FIG. 3). Chamber 221 is a pocket to contain and protect the duckbill protruding from the bottom of the bulb. The duckbill is located inside the pocket to a) help minimize residual formula left in the package after use; b) protect the duckbill from being deformed by outside forces, such as the operator's fingers, which could alter its functionality; and c) minimizes the overall size of the pump assembly.

The duckbill valve 210 may be integrally molded with the main body of the bulb 206, for example in a single multi-cavity molding operation, or may be attached as a separate molded component, depending on cost and tooling considerations. The duckbill valve 210 is oriented so that its slit 213 faces upward into a small pocket or recess region, such that liquid flowing through the slit enters the dosing chamber 206 and then is free to move around internal features of the bulb 206.

In alternative embodiments, the inlet one-way valve 320 may comprise any other one-way check valve, including but not limited to a flap valve, umbrella valve, ball-and-spring check valve, or other elastomeric or mechanical valve.

The bulb 206 is formed of a flexible elastomeric material having an elastic modulus of less than 100 megapascals (MPa) at room temperature. Suitable elastomers are polybutadienes, polyisoprenes, styrene-butadiene rubber, poly(styrene)-block-poly(butadiene), poly(acrylonitrile)-block-poly(styrene)-block-poly(butadiene) (ABS), polychloroprenes, epichlorohydrin rubber, polyacrylic rubber, silicone elastomers (polysiloxanes), fluorosilicone elastomers, fluoroelastomers, perfluoroelastomers, polyether block amides (PEBA), chlorosulfonated polyethylene, ethylene propylene diene rubber (EPR), ethylene-vinyl acetate elastomers, or the like, or a combination thereof.

Referring to FIGS. 3, 4A and 4B, the outlet one-way valve 220 is preferably an umbrella valve disposed within a rigid outlet module 231. The umbrella valve 219 comprises a flexible diaphragm portion that is normally seated against an outlet seat 203 to block fluid flow. When pressure in the dosing chamber 206 exceeds a threshold, the umbrella 219 lifts from its seat 203 and allows fluid to flow around the edges and out through an outlet passage.

In various embodiments, the outlet one-way valve 220 may comprise any suitable one-way valve, including an umbrella valve, a duckbill valve, a flap valve, a slit valve, a diaphragm valve, a ball check valve, a poppet valve (optionally spring biased), a pinch valve, a leaflet valve, or a cartridge-style check valve insert, and so on.

The outlet module 231 is configured to snap into the canoe 104. An annular seal region or barb 233 may be provided on the outlet module 231 so that, when pressed into a corresponding bore in the canoe structure, a secure mechanical and fluid-tight connection is formed. The outlet module 231 may include internal flow channels or slots 235 that direct fluid from the outlet side of the umbrella valve 219 the outlet tip or nozzle 108.

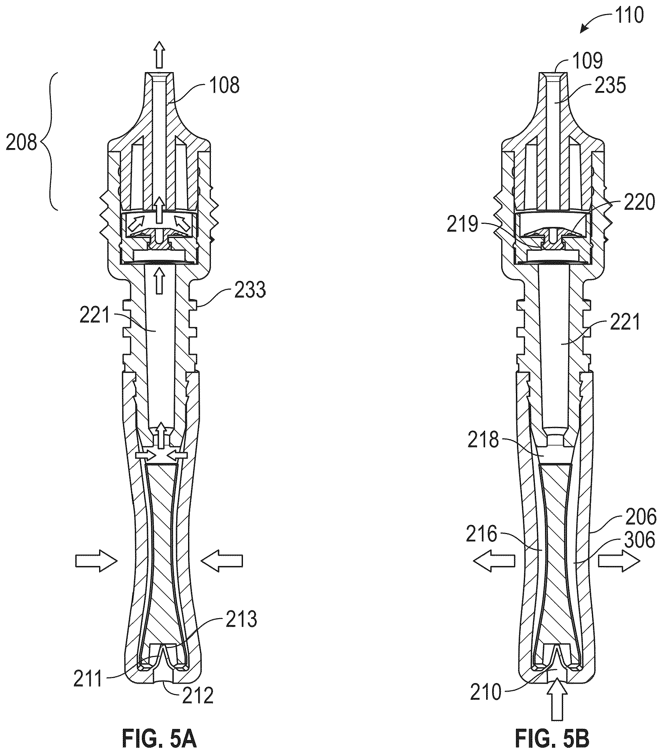

FIGS. 5A and 5B schematically illustrates the overall flow circuit. During a refill phase (See FIG. 5B), after the user releases pressure, the bulb 206 expands, creating negative pressure in the dosing chamber 221 relative to the collapsing pouch reservoir 120 (See FIG. 1A). This negative pressure causes the duckbill valve 210 to open, drawing liquid from reservoir 120 into the dosing chamber 206. At the same time, the umbrella valve 219 remains seated, so that no air is drawn in through the outlet. During a dispense phase, when the user squeezes the bulb 206 through the pouch 102, the dosing chamber 206 is compressed. The resulting positive pressure closes the duckbill valve 210 and opens the umbrella valve 219, forcing substantially the entire dosing volume out through the outlet tip 108 as a single drop or metered dose.

Because both valves are normally closed and are arranged in series with the dosing chamber, and because the flexible pouch 102 is collapsible, the dispensing assembly 100 operates as an airless diaphragm pump. The flexible pouch 102 gradually collapses as liquid is dispensed, and air is not drawn into the flexible pouch 102 during operation, which is advantageous for product stability.

The dispensing assembly 100 has a number of advantages. Because the bulb 206 is hidden inside the pouch 102 and canoe 104, the assembly 100 has an inherent level of child resistance. A child who does not know where to squeeze is less likely to actuate the device unintentionally. In commercial embodiments, an external printed squeeze indicator region or window may optionally be added to guide adult users.

The outlet tip or nozzle 108 may be removable or interchangeable. In one configuration, a relatively short, rigid nozzle is provided for ophthalmic use. In another configuration, a flexible, elongated tip 108 is coupled to the same outlet module and canoe 104, allowing comfortable insertion into a human or animal ear canal for otic administration. Other configurations are possible, including long, narrow tips for delivering lubricant into machinery, or luer-compatible tips for connection to other instruments.

Regardless of the tip geometry, one actuation of the bulb 206, defined by compressing the bulb 206 until it contacts the stem member 214, is designed to dispense about one metered dose. The system can be primed by several initial actuations to fill the dosing chamber 306, after which it dispenses consistent drops until the pouch is substantially exhausted.

The dispensing assembly can be manufactured in multiple ways. In one embodiment, the pouch is first formed and sealed on three sides, the canoe with pump assembly is attached, and the pouch is filled through the canoe end using standard fitment-filling equipment. After filling, the outlet tip is secured and the system is sealed and sterilized as required.

In another embodiment, the pouch is filled from an opposite end (e.g., a bottom tail), which is then heat-sealed after filling. This approach may be especially suitable for applications, such as cannabis or other non-sterile products, where sterility requirements are lower. The architecture also allows for potential pharmacy-level filling, wherein empty pouches and assemblies are provided to a pharmacy, which then fills and seals the pouch with patient-specific formulations.

The system is preferably single use/disposable in many medical applications, with the pouch and pump assembly being supplied pre-filled and sealed. However, in some embodiments the canoe 104 and pump assembly may be configured in a refillable manner, for example in conjunction with a reusable outer device that receives and seals to the pouch.

Because the internal components of the pump assembly are snap-fit together and because the pump assembly is heat-sealed as a unit to the pouch via the canoe structure, the number of seals and potential leak paths is significantly reduced compared to prior systems that use external actuators with multiple joints and interfaces. This simplified construction improves reliability, reduces cost, and allows for high evacuation of liquid; the collapsing pouch can evacuate substantially all of the liquid, leaving only a small residual volume at end of life.

The dispensing assembly is advantageous includes a flexible, collapsible pouch that serves as the primary reservoir for containing the liquid formulation. The pouch may be fabricated from one or more polymeric films, laminates, or multilayer barrier structures, such as polyethylene, polypropylene, EVOH-containing laminates, metallized films, biodegradable films, or any combination thereof, selected to provide appropriate mechanical strength, puncture resistance, moisture barrier performance, and chemical compatibility with the contained liquid. In preferred embodiments, the pouch is constructed from films thin enough to allow predictable manual deformation under typical squeezing forces, yet robust enough to withstand filling, sealing, sterilization, and distribution without rupture. Because the pouch collapses as liquid is dispensed, it permits airless operation by preventing air from being drawn into the internal pump assembly, thereby maintaining the integrity and sterility of the liquid product. The pouch may be pre-sterilized or sterilized post-fill, depending on regulatory requirements for the medication or fluid contained within.

In one embodiment, the dispensing assembly is configured to discharge a liquid formulation therethrough, wherein the liquid formulation is selected from fluids capable of flowing through a narrow discharge tip under modest manual actuation. Suitable liquids include aqueous liquids such as water, saline solutions, buffer solutions, and aqueous solutions of acids, bases, or pharmaceutically active agents; organic liquids including alcohols, ketones, esters, hydrocarbons, and aromatic solvents; oil-based liquids including mineral oils, silicone oils, essential oils, and vegetable oils; biological liquids including serum, plasma, cell culture media, and enzyme or protein solutions; and pharmaceutical, cosmetic, or industrial liquids including tinctures, syrups, liquid vitamins, fragrance compositions, dyes, inks, primers, and low-viscosity lubricants. In exemplary embodiments, the liquid is substantially free of suspended particulates and has a viscosity sufficiently low to permit controlled dropwise discharge through the dropper tip without clogging. The dropper may be formed from glass, polymeric, elastomeric, or fluoropolymer materials selected to be chemically compatible with the liquid to be dispensed, such that the dropper enables repeatable formation and release of discrete drops of the liquid during use.

While the invention has been described with reference to some embodiments, it will be understood by those skilled in the art that various changes may be made and equivalents may be substituted for elements thereof without departing from the scope of the invention. In addition, many modifications may be made to adapt a particular situation or material to the teachings of the invention without departing from essential scope thereof. Therefore, it is intended that the invention not be limited to the particular embodiments disclosed as the best mode contemplated for carrying out this invention, but that the invention will include all embodiments falling within the scope of the appended claims.

## Claims

1. A dispensing assembly, comprising:
a flexible, collapsible pouch defining a reservoir configured to contain a liquid;
a rigid canoe sealed to the pouch and extending into an interior of the pouch;
a squeezable bulb disposed at least partially within the pouch and supported by the canoe structure, the bulb defining a dosing chamber;
a first one-way valve fluidly connecting the reservoir to the dosing chamber;
a second one-way valve fluidly connecting the dosing chamber to an outlet; and
an outlet tip in fluid communication with the second one-way valve;
wherein the pouch and the squeezable bulb are configured such that application of external pressure through the pouch collapses the squeezable bulb to expel a metered dose of liquid from the dosing chamber through the second one-way valve and the outlet tip, and release of the external pressure allows the bulb to expand and refill the dosing chamber from the reservoir through the first one-way valve while the second one-way valve remains closed, thereby maintaining an airless condition within the pouch.

2. The dispensing assembly of claim 1, wherein the first one-way valve comprises a duckbill valve having opposed lips defining a slit that opens under negative pressure in the dosing chamber and closes under positive pressure in the dosing chamber; and wherein the duckbill valve is integrally molded with a body of the squeezable bulb as a single elastomeric component.

3. The dispensing assembly of claim 1, wherein the second one-way valve comprises an umbrella valve disposed in a rigid outlet module, the umbrella valve being configured to lift from a seat under positive pressure in the dosing chamber and to remain seated under negative pressure in the dosing chamber; and wherein the rigid outlet module is snap-fit into the canoe structure and includes an annular seal configured to form a fluid-tight connection with the canoe structure.

4. The dispensing assembly of claim 1, further comprising an internal stem member disposed within the squeezable bulb, the stem member having a contour configured to limit a collapse of a wall of the squeezable bulb, thereby defining a predetermined dosing volume of the dosing chamber; and wherein the contour of the stem member approximates two forks of a tuning prong, such that the squeezable bulb collapses against the stem member and wherein a metered dose is dispensed upon a full collapse of the squeezable bulb against the stem member.

5. The dispensing assembly of claim 1, wherein the canoe structure comprises a molded plastic fitment having a canoe-like cross-section and at least one rib configured to support and position the squeezable bulb and the second one-way valve within the pouch.

6. The dispensing assembly of claim 1, wherein the pouch comprises a film laminate heat-sealed to a flange of the canoe structure, and wherein no adhesive or weld is used between the bulb, the first one-way valve, the second one-way valve, and the outlet tip.

7. The dispensing assembly of claim 1, wherein the reservoir and dosing chamber are configured such that substantially all of the liquid in the pouch is dispensed as the pouch collapses, with only a small residual volume remaining at end of life.

8. The dispensing assembly of claim 1, wherein the outlet tip is removably coupled to the outlet and is selected from a group consisting of a short ophthalmic nozzle, a flexible otic tip, and an elongated industrial lubricant tip; and wherein the outlet tip comprises a flexible elongate member configured to be inserted into an ear canal of a human or animal.

9. The dispensing assembly of claim 1, wherein the pouch includes an externally visible indicator region or window aligned with the bulb, the indicator region providing a visual cue regarding where to apply external pressure.

10. The dispensing assembly of claim 1, wherein the squeezable bulb is formed of a silicone or thermoplastic elastomer and the canoe structure is formed of a thermoplastic selected from polyethylene, polypropylene, or blends thereof.

11. The dispensing assembly of claim 1, wherein the first one-way valve is configured to open in response to negative pressure generated in the dosing chamber upon expansion of the bulb, and the second one-way valve is configured to open in response to positive pressure generated in the dosing chamber upon collapse of the bulb, such that the valves operate oppositely under the same pressure conditions.

12. The dispensing assembly of claim 1, wherein the canoe structure is configured to receive a filling nozzle during manufacturing such that the pouch is filled through the canoe structure prior to installation of the outlet tip and wherein the pouch and canoe structure together provide an inherent child-resistant feature by enclosing the bulb such that the bulb is not visible and is not directly accessible from outside the pouch.

13. The dispensing assembly of claim 1, wherein the first one-way valve comprises a separate check valve disposed at an end of a flexible tube that is coupled to the bulb and wherein at least one of the first one-way valve and the second one-way valve is an elastomeric valve selected from the group consisting of duckbill valves, umbrella valves, and flap valves; and wherein internal flow channels are provided between the dosing chamber and the second one-way valve so that liquid continues to flow around the stem member and through the channels during collapse of the bulb without being blocked by contact regions of the bulb and stem.

14. A method of dispensing a liquid, comprising:
providing a dispensing assembly according to claim 1, the pouch being pre-filled with a liquid in the reservoir;
applying external pressure to a region of the pouch overlying the squeezable bulb to collapse the bulb against an internal stem member, thereby generating positive pressure in the dosing chamber and opening the second one-way valve while closing the first one-way valve, so as to expel substantially one metered dose of the liquid from the outlet tip; and
releasing the external pressure to allow the bulb to expand, thereby generating negative pressure in the dosing chamber and opening the first one-way valve while closing the second one-way valve, so as to refill the dosing chamber from the reservoir while preventing air ingress through the outlet tip.

15. A system for delivering metered doses of a liquid, comprising:
a dispensing assembly according to claim 1, pre-filled with a liquid selected from a medication, a lubricant, a biological fluid, a pharmaceutical liquid, an aqueous fluid, an organic fluid, or a combination thereof; and
instructions indicating that the user is to apply external pressure to a specified region of the pouch to deliver one metered drop per actuation until the pouch collapses.
